# EUROPEAN PATENT APPLICATION

(11) **EP 3 587 402 A1**
(43) Date of publication of application: **01.01.2020**
(21) Application number: 19020441.2
(22) Date of filing: 07.04.2015
(51) Int. Cl.: C07D 233/86

(54) **PROCESS FOR PRODUCING ENZALUTAMIDE**

(30) Priority: 07.04.2014 CZ 20140232
(62) Divisional of application: 15720253.2
(71) Applicant: Zentiva K.S., 102 37 Praha 10 (CZ)
(72) Inventor: Stach, Jan, 190 16 Praha 9 (CZ); Klecan, Ondrej, 282 01 Cesky Brod (CZ); Lehnert, Petr, 106 00 Praha 10 (CZ); Rymes, Jan, 500 02 Hradec Kralove (CZ)
(74) Representative: Jirotkova, Ivana

(57) **Abstract**

The application relates to a process for producing enzalutamide of formula I, starting from the intermediate of formula XI, which cyclizes with the isothiocyanate of formula VIII in the presence of a base in a suitable solvent.

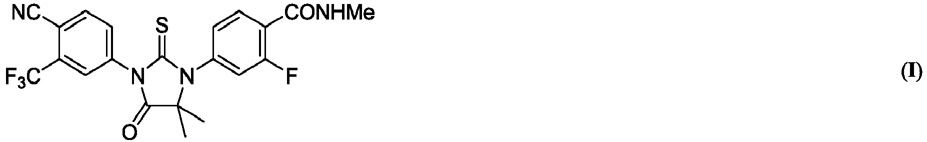

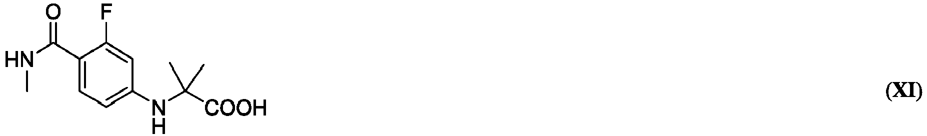

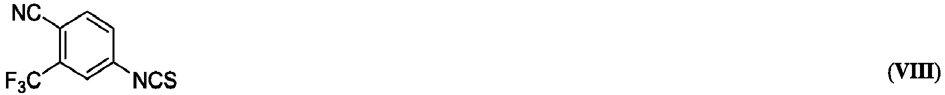

## Description

### Technical Field

The invention relates to a new process for producing the drug enzalutamide (I), 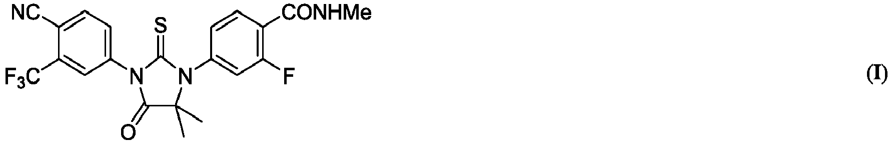 which is used for the treatment of prostate cancer. Enzalutamide (formerly referred to as MDV3100) is a completely new drug that acts as an inhibitor of signaling of androgen receptors (AR) - unlike the other currently available anti-androgens it prevents translocation of AR to the cell nucleus, their binding to DNA and the intake of other transcription activators; it is also characterized with a higher affinity to AR. In pre-clinical studies its ability to induce tumor reduction has also been proved.

### Background Art

Synthesis of enzalutamide (Scheme 1) was first described in the basic patent application of 2006 (WO2006/124118). The key step is the final cyclization of the isothiocyanate **VIII** and nitrile **IX**, which, however, provides a very low yield.

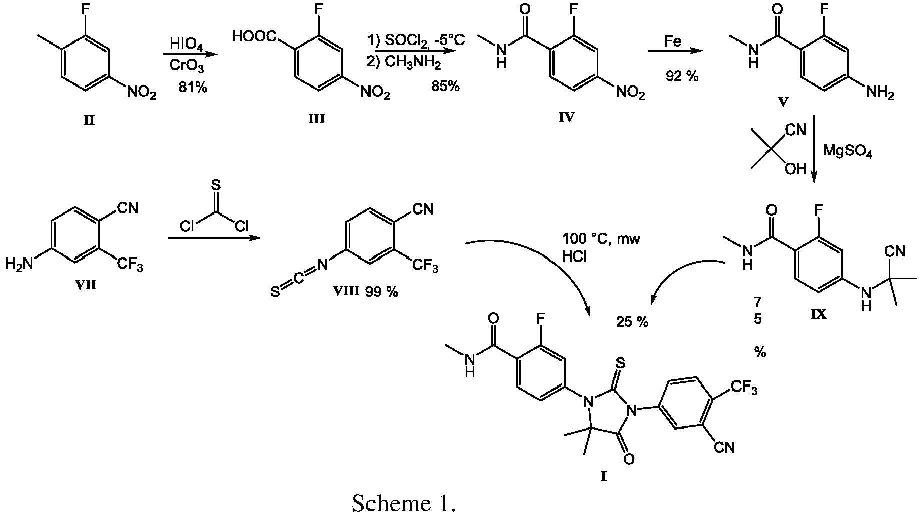

A new process of the company Medivation Prostate Therapeutics, Inc. (WO2011/106570) improves the final cyclization by using the methyl ester **X** for the cyclization, which is prepared from the starting acid **XI** (Scheme 2).

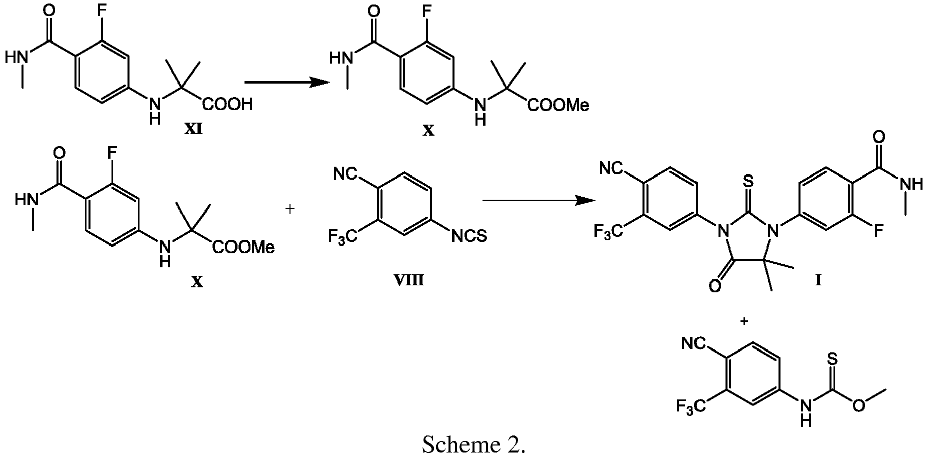

The patent application mentions that the residual bases (e.g. inorganic ions) have a negative impact on the cyclization reaction and lead to a number of side reactions. The reaction is carried out at an elevated temperature by heating in a mixture of DMSO and isopropyl acetate. However, the application does not specify in particular what side reactions occur during the cyclization. This improved method provides a good yield in the last step; however, it requires a 100% by weight excess of the isothiocyanate of formula **VIII**, which reacts with methanol released during the cyclization reaction. In a repeated exact reproduction of the above mentioned procedure of this application enzalutamide was always isolated with an impurity of 1% by weigh of the respective methyl thiocarbamate (Scheme 2). Thus, from the economical and industrial point of view this method is not quite efficient. The above mentioned method involves transformation of the acid of formula **XI** to the ester of formula **X** with methyl iodide, which is toxic (strong alkylating agent), which complicates industrial production. The authors apparently also considered the possibility of carrying out the cyclization directly with the acid of formula **XI.** However, this reaction only led to a negligible yield of 2% by weight. The intermediate that was used for this reaction was the diacid of formula **XII.** The scheme of this reaction is shown below (Scheme 3). However, since the reaction was carried out in ethanol, in fact ethanol apparently reacted first with the isothiocyanate of formula **VIII** and it was only the subsequently produced thiocarbamate which reacted with the diacid of formula XII, providing the product of formula XIII in a scant yield.

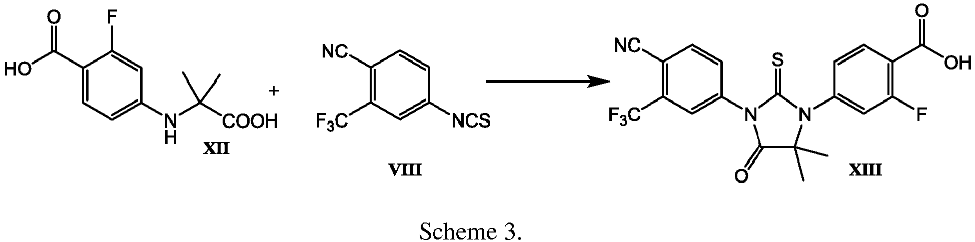

### Disclosure of Invention

The invention provides a new process for producing enzalutamide of formula I, 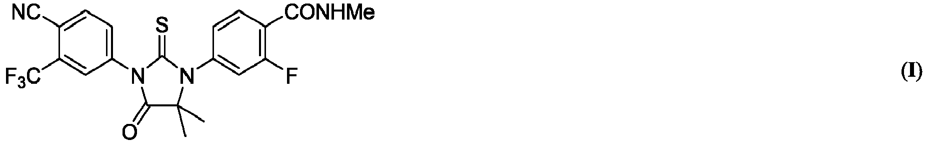 starting from the intermediate of formula XI, 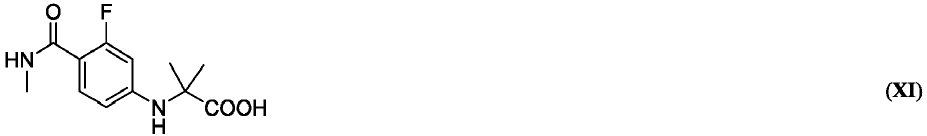 which cyclizes with the isothiocyanate of formula VIII 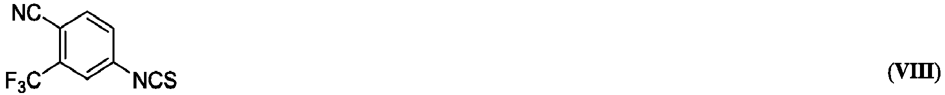 in the presence of a base, an alcohol of the general formula R-OH as an additive, in a suitable solvent, wherein R is an alkyl with the number of carbon atoms C₁-C₂₀, or a substituted alkyl with the number of carbon atoms C₁-C₂₀, an aryl with the number of carbon atoms C₆-C₁₆, or a substituted aryl with the number of carbon atoms C₆-C₁₆.

The essence of the invention is the surprising effect of the added R-OH alcohol that prevents side reactions. In addition, the process is one step shorter than that described in the patent application WO2011/106570. The yield of our - one step shorter - process is about 75%. Another indisputable advantage of our method is the fact that there is no need to use the toxic methyl iodide and that the consumption of the isothiocyanate of formula **VIII** is lower.

Initial studies dealing with the development of the cyclization stage showed that under a great deal of conditions used two most important impurities of formulae **XIV** and **XV** were produced. The impurity of formula **XV** - "oxo" enzalutamide - is very similar to enzalutamide and it also has almost the same physical - chemical characteristics, which makes this impurity difficult to remove from its mixtures with enzalutamide with the use of common industrial methods, e.g. crystallization.

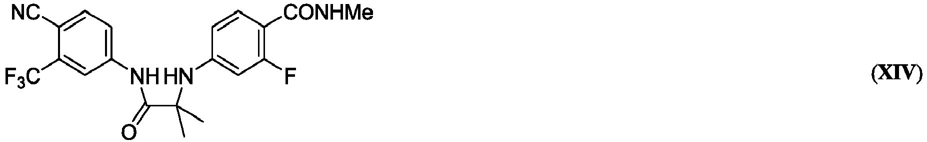

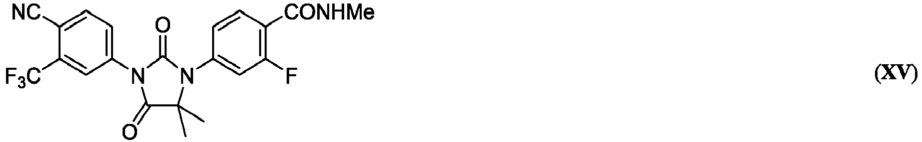

The amount of the impurity of formula **XV** commonly varied in the range from 1 to 20 % by weight in the crude reaction mixture depending on carrying out the reaction **without addition of an alcohol.** With regard to the limit for known impurities, which is 0.15 % by weight for an active pharmaceutical ingredient, a substance prepared this way cannot be used for the preparation of the final drug form. Also, the base that was mentioned in the patent application WO2011/106570 as one of the sources of side reactions is always necessary for the cyclization reaction.

However, in one of the experiments conducted in chloroform only 0.2% by weight of this impurity was surprisingly found. A closer examination showed that the cause of the hopeful result was residual ethanol, which is commonly used in chloroform as a stabilizer.

Another comparative experiment conducted in trifluorotoluene (TFT) showed that while cyclization in TFT alone led to formation of 1.3 % by weight of "oxo" enzalutamide in the reaction mixture, cyclization in TFT with the addition of 1% by weight of ethanol only led to the production of 0.3% by weight of "oxo" enzalutamide.

Further optimization brought the finding that to reduce the amount of produced "oxo" enzalutamide to the limit of 0.2% by weight, several equivalents of ethanol were necessary, which however caused other complications during processing as ethanol reacts with the isothiocyanate of formula **VIII**, producing the thiocarbamate of formula **XVI** (Scheme 4).

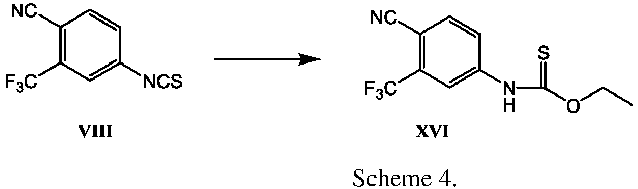

Further investigation surprisingly revealed that the most suitable alcohol was an aromatic phenol, which reduces the formation of "oxo" enzalutamide even more (down to the limit of 0.1% by weight). In addition, phenol does not react with the isothiocyanate of formula **VIII** to produce the carbamate and therefore there is no need to have a high excess of the substance of formula **VIII** for the cyclization. Further investigation of the cyclization process showed that the best results could be achieved with the use of ethyl diisopropylamine (EDIPA) as the base. This combination (phenol + EDIPA) apparently has a synergistic effect as the base itself does not have any impact on reduction of "oxo" enzalutamide production.

The results of the screening are summarized in table I below.

**Table I**

| **Exp**. | **Solvent** | **Alcohol** | **Base** | **% by weight Oxo imp.** |
|---|---|---|---|---|
| ENZ-123A | TFT | MeOH | TEA | 1.87 |
| ENZ-123B | TFT | t-BuOH | TEA | 1.44 |
| ENZ-123C | TFT | Benzyl alcohol | TEA | 1.38 |
| ENZ-123D | TFT | Phenol | TEA | 0.86 |
| ENZ-123E | Toluene | MeOH | TEA | 1.67 |
| ENZ-123F | Toluene | t-BuOH | TEA | 1.27 |
| ENZ-123G | Toluene | Benzyl alcohol | TEA | 1.16 |
| ENZ-123H | Toluene | Phenol | TEA | 0.69 |
| ENZ-127A | TFT | Phenol | TEA | 0,29 |
| ENZ-127B | TFT | Phenol | EDIPA | 0.11 |
| ENZ-127C | TFT | Phenol | Pyridine | 4.02 |
| ENZ-127D | TFT | Phenol | 2,6-Lutidine | 0.91 |
| ENZ-130B | TFT | BHT | TEA | 1.85 |

The cyclization in accordance with the present invention is carried out at the temperatures of from 20°C to 140°C, in a preferred embodiment a temperature in the range of 40°C to 60°C is used. As the base a tertiary amine is used, in a preferred embodiment triethylamine or EDIPA. Suitable solvents for the cyclization comprise mainly toluene, α,α,α-trifluorotoluene (TFT), tetrahydrofuran, in a preferred embodiment TFT is used. The amount of the ROH alcohol in the reaction is 0.1 to 100 molar equivalents related to the starting substance of formula XI. As the added alcohol that is necessary to reduce the content of "oxo"enzalutamide an alcohol of the general formula ROH can be used, wherein R is an alkyl with the number of carbon atoms C₁-C₂₀, or a substituted alkyl with the number of carbon atoms C₁-C₂₀, an aryl with the number of carbon atoms C₆-C₁₆, or a substituted aryl with the number of carbon atoms C₆-C₁₆. In a preferred embodiment EtOH, phenol, or *ortho, meta* or para-cresol is used. If an alcohol of the ROH type is used, wherein R is an alkyl with the number of carbon atoms C₁-C₂₀, or a substituted alkyl with the number of carbon atoms C₁-C₂₀, in a preferred embodiment 1 to 5 molar equivalents of the alcohol are used related to the starting substance of formula **XI.** If an aromatic alcohol of the ROH type is used, wherein R is an aryl with the number of carbon atoms C₆-C₁₆, or a substituted aryl with the number of carbon atoms C₆-C₁₆, in a preferred embodiment this alcohol is used in an amount of 0.1 to 20 molar equivalents of the alcohol related to the starting substance of formula **XI.** In another embodiment an aromatic alcohol can also be directly used as the solvent. In such a case in a preferred embodiment phenol, *ortho, meta* or *para*-cresol is used as the solvent. Enzalutamide obtained this way can be re-crystallized from suitable solvents, methanol or isopropanol being especially suitable.

The invention is elucidated in a more detailed way in the examples below. These examples, which illustrate the improvement of the procedure in accordance with the invention, only have an illustrative character and do not restrict the scope of the invention in any respect.

The present description discloses the following embodiments:
1) A process for producing enzalutamide of formula **I** 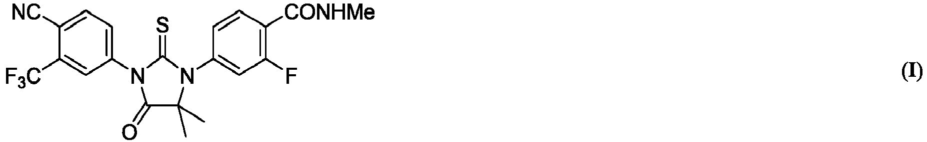 characterized in that the intermediate of formula XI 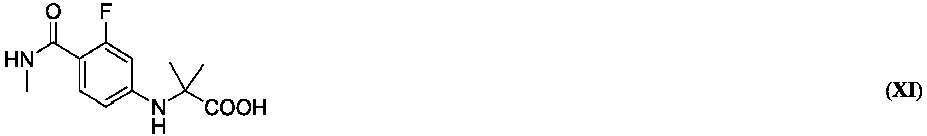 is reacted with the intermediate of formula VIII 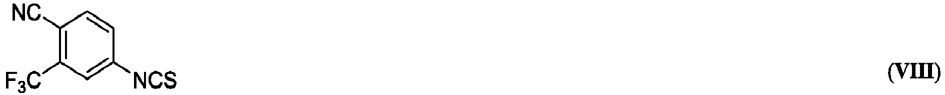 in a suitable solvent, in the presence of a base and an alcohol of formula R-OH, wherein R is an alkyl with the number of carbon atoms C₁-C₂₀, or a substituted alkyl with the number of carbon atoms C₁-C₂₀, an aryl with the number of carbon atoms C₆-C₁₆, or a substituted aryl with the number of carbon atoms C₆-C₁₆.
2) The process according to embodiment 1, characterized in that an aromatic alcohol is used as the alcohol.
3) The process according to embodiments 1 or 2, characterized in that the aromatic alcohol is selected from the group including phenol, *ortho, meta* or *para*-cresol, preferably phenol.
4) The process according to embodiment 1, characterized in that the base used is a tertiary amine, preferably triethylamine or ethyl diisopropylamine.
5) The process according to embodiment 1, characterized in that the reaction is carried out at a temperature of from 20°C to 140°C; preferably at a temperature of from 40°C to 60°C.
6) The process according to embodiment 1, characterized in that the suitable solvent is toluene, α,α,α-trifluorotoluene, tetrahydrofuran, preferably α,α,α-trifluorotoluene.
7) The process according to embodiments 1 or 6, characterized in that said suitable solvent and at the same time said alcohol of formula ROH, wherein R is as defined in claim 1, is an alcohol of formula ROH.
8) The process according to embodiment 7, characterized in that said suitable solvent and at the same time said alcohol of formula ROH is an aromatic alcohol, preferably phenol, *ortho, meta* or *para*-cresol.
9) The process according to embodiment 1, characterized in that the amount of the ROH alcohol in the reaction is 0.1 to 100 molar equivalents related to the starting substance of formula **XI.**
10) The process according to embodiments 1 or 9, characterized in that the ROH alcohol, wherein R is an alkyl or substituted alkyl, is used in an amount of 1 to 5 molar equivalents of the alcohol related to the starting substance of formula **XI.**
11) The process according to embodiments 1 or 9, characterized in that the ROH alcohol, wherein R is an aryl or substituted aryl, is used in an amount of 0.1 to 20 molar equivalents of the alcohol related to the starting substance of formula **XI.**
12) The process according to any one of the preceding embodiments, characterized in that enzalutamide is further crystallized from a suitable solvent, preferably methanol or isopropanol.

### Examples

### Example 1

### 2-Fluoro-4-nitro-N-methylbenzamide of formula IV.

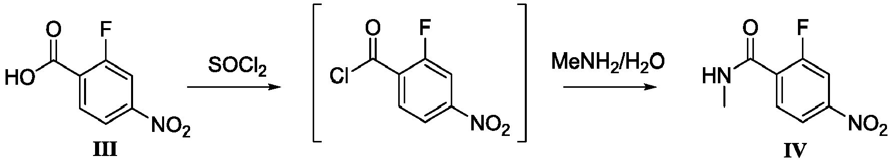

2000 ml of isopropyl acetate, 10 ml of DMF were added to 250 g (1.35 mol) of 2-fluoro-4-nitrobenzoic acid of formula **III** in a 5000-ml reactor, the mixture was heated up to 60°C and the vessel was thoroughly rinsed with nitrogen. Then, 193 g (1.62 mol) of thionyl chloride was added dropwise within 50 min and the mixture was further stirred at 60°C overnight. The next day the mixture was cooled down to -10°C, the turbidity was filtered off through a folded filter and the obtained clear solution of 2-fluoro-4-nitrobenzoic acid chloride was transferred into a bottle and kept under an inert nitrogen atmosphere.

500 ml of isopropyl acetate, 524 g of 40% aqueous methylamine were charged into a reaction vessel and the mixture was cooled down to 0°C. A solution of 2-fluoro-4-nitrobenzoic acid chloride was added dropwise to this mixture within 4 hours and the resulting mixture was stirred for another 1 h. The separated solid product was filtered off on a Büchner funnel, washed with a sizeable amount of water and air-dried.

241 g (90% by weight) of the product of formula **IV** was obtained in the form of yellow crystals, melt. point 161 to 164°C. HPLC purity: 99.83%. ¹H NMR (CDCl₃): δ(ppm): 3.10 (dd, 3H), 6.8 (b, 1H), 8.03 (dd, 1H), 8.14 (dd, 1H), 8.33 (m, 1H).

### Example 2

### 4-Amino-2-fluoro-N-methylbenzamide hydrochloride of formula V.

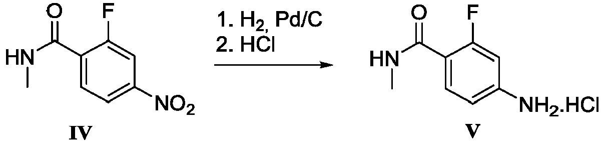

40.0 g (0.202 mol) of 2-fluoro-4-nitro-N-methylbenzamide of formula **IV** was charged into a 1500 ml autoclave and further 500 ml of methanol and 0.50 g of 10% (by weight) palladium on active carbon were added. The mixture was hydrogenated at the pressure of 600 kPa and temperature of 50°C for 1 h, then the catalyst was removed by filtration through a kieselguhr layer and the solvent was evaporated until dry. The evaporation product (35.6 g) was dissolved in 180 ml of hot ethanol and 21 g of concentrated (35%) hydrochloric acid (0.202 mmol) was added. After seeding and cooling the separated solid product was aspirated and washed with ethanol. 29,3 g (71% by weight) of the product of formula **V** was obtained in the form of white crystals, melt. point 222 to 228°C (decomposition). HPLC purity: 99.89%. ¹H NMR (DMSO): δ(ppm): 2.75 (d, 3H), 6.80 (m, 2H), 7.57 (t, 1H), 7.96 (b, 1H), 8.50 (b, 3H).

### Example 3

### 2-[3-Fluoro-4-(methylcarbamoyl)phenylamino]-2-methylpropanoic acid of formula XI.

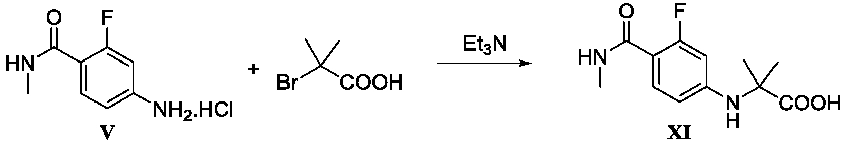

50.0 g (0.244 mol) of 4-amino-2-fluoro-N-methylbenzamide hydrochloride of formula **V** was charged into a 500-ml vessel equipped with an anchor agitator, 50 ml of dimethylacetamide was added and the mixture was heated up to 70°C. Then, 68.0 g (0.672 mol) of triethylamine was added and the mixture was stirred for 60 min. After that the temperature was increased to 100°C and a pre-heated solution of 54.3 g (0.325 mol) of 2-bromo-2-methylpropionic acid in 25 ml of dimethylacetamide was added dropwise to the mixture during 10 min. The mixture was agitated at 100°C for another 1 ½ h, then it was cooled down to 40°C and 125 ml of water and a solution of 40 g of citric acid in 100 ml of water were added. The mixture was seeded with the product, cooled down to 20°C and stirred overnight. The separated product was aspirated on a Büchner funnel, washed with a sizeable amount of water and dried in a vacuum drier overnight. 33,2 g (53% by weight) of the product of formula **XI** was obtained in the form of pinkish crystals, melt. point 205 to 210°C. HPLC purity: 97.46%. ¹H NMR (DMSO): δ(ppm): 1.45 (s, 6H), 2.73 (d, 3H), 6.16 (dd, 1H), 6.35 (dd, 1H), 6.70 (b, 1H), 7.46 (t, 1H), 7.65 (t, 1H), 12.7 (b, 1H).

### Example 4

### Enzalutamide of formula I

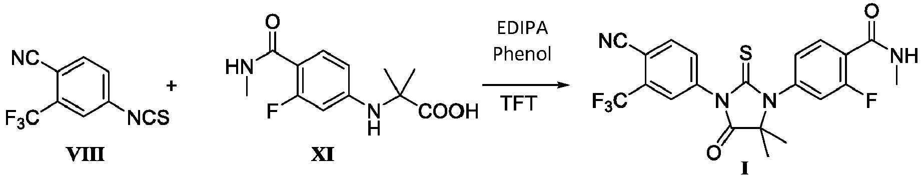

50 ml of α,α,α-trifluorotoluene was added to 10.0 g (39.3 mmol) of 2-[3-fluoro-4-(methylcarbamoyl)phenylamino]-2-methylpropanoic acid of formula XI and the mixture was heated up to 60°C. Then, 5.1 g (39.3 mmol) of ethyl diisopropylamine and 11.1 g (118 mmol) of phenol were added and the flask was rinsed with nitrogen. Subsequently, a solution of 13.5 g (59.0 mmol) 4-isothiocyanato-2-(trifluoromethyl)benzonitrile of formula **VIII** in 30 ml of α,α,α-trifluorotoluene was added during 20 min and the mixture was stirred at 60°C for another 1 ¼ h. Then, 150 ml of ethyl acetate and 150 ml of water were added, the organic phase was separated, washed with 200 ml of 3% hydrochloric acid and evaporated until dry. The evaporation product was re-crystallized from 50 ml of isopropyl alcohol with the addition of 0.5 ml of concentrated HCl; 13.7 g (75% by weight) of the crystalline substance of formula **I** was obtained. Melt. point 197 to 199°C. HPLC purity: 99.76%, 0.08% of the oxo impurity of formula XV. ¹H NMR (CDCl₃): δ(ppm): 1.64 (s, 6H), 3.10 (t, 3H), 6.75 (b, 1H), 7.17 (dd, 1H), 7.27 (dd, 1H), 7.85 (dd, 1H), 7.97 (d, 1H), 8.01 (d, 1H), 8.30 (t, 1H)

### Example 5

### Enzalutamide of formula I

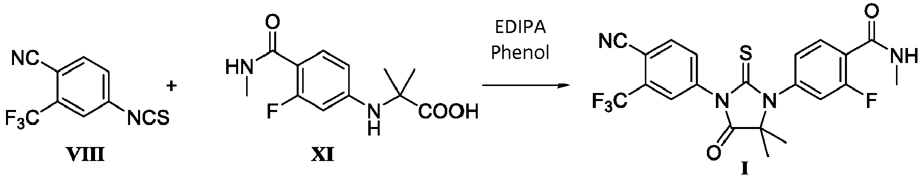

22.2 g (236 mmol) of phenol, 9.15 g (40 mmol) of 4-isothiocyanato-2-(trifluoromethyl)benzonitrile of formula **VIII**, 5.1 g (39.3 mmol) of ethyl diisopropylamine were added to 10.0 g (39.3 mmol) of 2-[3-fluoro-4-(methylcarbamoyl)phenylamino]-2-methylpropanoic acid of formula **XI** and the mixture was heated up to 60°C for 4 h. Then, 150 ml of ethyl acetate and 150 ml of water were added, the organic phase was separated, washed with a 200 ml of a 3% (by weight) solution of potassium carbonate in water, a 3% solution of hydrochloric acid in water, and evaporated until dry. The evaporation product was re-crystallized from 50 ml of isopropyl alcohol with the addition of 0.5 ml of concentrated HCl; 14.1 g (77 % by weight) of the crystalline substance of formula **I** was obtained.

### Example 6

### Enzalutamide of formula I

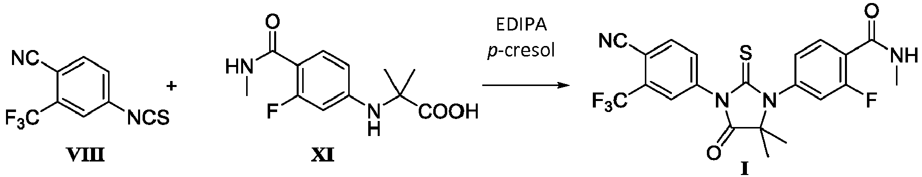

40 ml of *p*-cresol, 9.15 g (40 mmol) of 4-isothiocyanato-2-(trifluoromethyl)benzonitrile of formula **VIII**, 5.1 g (39.3 mmol) of ethyl diisopropylamine were added to 10.0 g (39.3 mmol) of 2-[3-fluoro-4-(methylcarbamoyl)phenylamino]-2-methylpropanoic acid of formula **XI** and the mixture was heated up to 60°C for 4 h. Then, 150 ml of ethyl acetate and 150 ml of water were added, the organic phase was separated, washed with 200 ml of a 3% solution of potassium carbonate in water, a 3% solution of hydrochloric acid in water and evaporated until dry. The evaporation product was re-crystallized from 50 ml of isopropyl alcohol with the addition of 0.5 ml of concentrated HCl; 12.0 g (65% by weight) of the crystalline substance of formula **I** was obtained.

## Claims

1. A process for producing enzalutamide of formula **I** 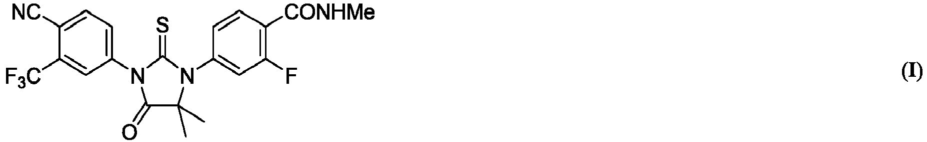 **characterized in that** the intermediate of formula XI 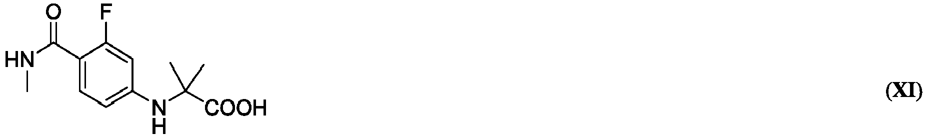 is reacted with the intermediate of formula VIII 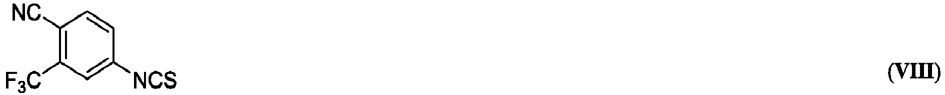 in a suitable solvent, in the presence of a base.

2. The process according to claim 1, **characterized in** the presence of an alcohol of formula R-OH, wherein R is an alkyl with the number of carbon atoms C₁-C₂₀, or a substituted alkyl with the number of carbon atoms C₁-C₂₀, an aryl with the number of carbon atoms C₆-C₁₆, or a substituted aryl with the number of carbon atoms C₆-C₁₆.

3. The process according to claim 2, **characterized in that** an aromatic alcohol is used as the alcohol.

4. The process according to claim 3, **characterized in that** the aromatic alcohol is selected from the group including phenol, *ortho, meta* or *para*-cresol, preferably phenol.

5. The process according to claim 1, **characterized in that** the base used is a tertiary amine, preferably triethylamine or ethyl diisopropylamine.

6. The process according to claim 1, **characterized in that** the reaction is carried out at a temperature of from 20°C to 140°C; preferably at a temperature of from 40°C to 60°C.

7. The process according to claim 1, **characterized in that** the suitable solvent is toluene, α,α,α-trifluorotoluene, tetrahydrofuran, preferably α,α,α-trifluorotoluene.

8. The process according to claims 2 or 7, **characterized in that** said suitable solvent and at the same time said alcohol of formula ROH, wherein R is as defined in claim 1, is an alcohol of formula ROH.

9. The process according to claim 8, **characterized in that** said suitable solvent and at the same time said alcohol of formula ROH is an aromatic alcohol, preferably phenol, *ortho, meta* or *para*-cresol.

10. The process according to claim 2, **characterized in that** the amount of the ROH alcohol in the reaction is 0.1 to 100 molar equivalents related to the starting substance of formula **XI.**

11. The process according to claims 2 or 10, **characterized in that** the ROH alcohol, wherein R is an alkyl or substituted alkyl, is used in an amount of 1 to 5 molar equivalents of the alcohol related to the starting substance of formula **XI.**

12. The process according to claims 2 or 10, **characterized in that** the ROH alcohol, wherein R is an aryl or substituted aryl, is used in an amount of 0.1 to 20 molar equivalents of the alcohol related to the starting substance of formula **XI.**

13. The process according to any one of the preceding claims, **characterized in that** enzalutamide is further crystallized from a suitable solvent, preferably methanol or isopropanol.
